# EUROPÄISCHE PATENTSCHRIFT

(11) **EP 0 379 161 B1**
(45) Veröffentlichungstag und Bekanntmachung des Hinweises auf die Patenterteilung: **21.09.1994**
(21) Anmeldenummer: 90100887.0
(22) Anmeldetag: 17.01.1990
(51) Int. Cl.: C08G 73/04, A61K 31/785

(54) **Verwendung alkylierter Polyethylenimine als gallensäureadsorbierende Arzneimittel sowie pharmazeutische Präparate**
Use of alkylated polyethyleneimines as therapeutic agent adsorbing bile acid and pharmaceutical compositions thereof
Utilisation des dérivés alcoylés de polyéthylèneimine comme médicaments adsorbants d'acide biliaire et compositions pharmaceutiques

(30) Priorität: 20.01.1989 DE 3901527
(43) Veröffentlichungstag der Anmeldung: 25.07.1990
(73) Patentinhaber: HOECHST AKTIENGESELLSCHAFT, 65926 Frankfurt am Main (DE)
(72) Erfinder: Heitz, Walter, Prof. Dr., D-3570 Kirchhain (DE); Fischer, Thomas, D-3550 Marburg (DE); Kerekjarto, Bela, Dr., D-6238 Hofheim am Taunus (DE)

(56) Entgegenhaltungen:
- DE-A- 2 946 026
- US-A- 3 251 778
- US-A- 3 332 841
- PATENT ABSTRACTS OF JAPAN, Band 4, Nr. 85 (C-15)(567),18. Juni 1980; & JP - A - 5548221
- PATENT ABSTRACTS OF JAPAN, Band 12, Nr. 122 (C-488)(2969), 15. April 1988; & JP - A - 62243620

## Beschreibung

Die Erfindung betrifft die Verwendung alkylierter Polyethyleniminderivate als Arzneimittel, insbesondere zur Senkung erhöhter Lipidspiegel, sowie pharmazeutische Zubereitungen auf Basis dieser Verbindungen.

Unlösliche, basische, vernetzte Polymere werden seit geraumer Zeit zur Bindung von Gallensäure verwendet und aufgrund dieser Eigenschaften therapeutisch genutzt. Als Therapieobjekt werden die chologene Diarrhoe (z.B. nach Ileumresektion) und erhöhter Cholesterin-Blutspiegel kausal behandelt. Im letzteren Fall handelt es sich um den Eingriff in den enterohepatischen Kreislauf, wobei anstelle des aus dem Kreislauf genommenen Gallensäureanteils die entsprechende Neusynthese aus Cholesterin in der Leber provoziert wird. Zur Deckung des Cholesterinbedarfs in der Leber wird auf das zirkulierende LDL (Low Density Lipoprotein)-Cholesterin zurückgegriffen, wobei die hepatischen LDL-Rezeptoren in vermehrter Anzahl zur Wirkung kommen. Die so erfolgte Beschleunigung des LDL-Katabolismus wirkt sich durch die Herabsetzung des atherogenen Cholesterinanteils im Blut aus.

Die als Arzneimittel in der Verwendung befindlichen Ionenaustauscher besitzen als aktive Gruppen entweder quartäre Ammoniumgruppen (wie Colestyramin) oder sekundäre bzw. tertiäre Amingruppen (wie Colestipol). Die Tagesdosis an Colestyramin beträgt zweckmäßigerweise 12 - 24 g, als Tageshöchstdosis werden 32 g empfohlen. 15 - 30 g ist die empfohlene tägliche Colestipol-Dosis. Geschmack, Geruch und hohe Dosierung erschweren die Patienten-Compliance. Die Nebenwirkungen gehen auf mangelnde Selektivität (z.B. Avitaminosen) zurück, die auch bei der Dosierung simultan gegebener Medikamente berücksichtigt werden müssen, aber auch auf Gallensäureverarmung, die verschiedene gastrointestinale Störungen (Obstipation, Steatorrhoe) unterschiedlichen Grades hervorrufen. Für beide Präparate wurde eine therapeutische Bedeutung durch Kombination mit anderen hypolipidämisch wirkenden Pharmaka wie Fibrate, HMG-CoA-Reduktase-Inhibitoren, Probucol (vgl. z.B. M.N. CAYEN, Pharmac. Ther. 29, 187 (1985) und 8th International Symposium on Atherosclerosis, Rome, Oct. 9-13, 1988, Abstracts S. 544, 608, 710) beschrieben, wobei die erzielten Effekte auch die Therapie von schweren Hyperlipidämien ermöglichen. Deshalb erscheint es bedeutungsvoll, bei dem gegebenen Wirkprinzip geeignete Stoffe ohne die Nachteile der gegenwärtig verwendeten Präparate zu finden.

Folgende Merkmale der genannten Präparate und insbesondere von Colestipol sind als verbesserungswürdig anzusehen:
1. Die hohen Tagesdosen, die zurückzuführen sind auf eine relativ geringe Bindungsrate bei neutralem pH in isotonem Medium und der (teilweisen) Wiederfreisetzung der adsorbierten Gallensäure.
2. Die qualitative Verschiebung der Gallensäurezusammensetzung der Galle mit abnehmender Tendenz für Chenodesoxycholsäure und die damit verbundene zunehmende Gefahr für Cholelithiasis.
3. Das Fehlen einer dämpfenden Wirkung auf den Cholesterinstoffwechsel der Darmbakterien.
4. Die zu hohe Bindungsrate von Vitaminen und Pharmaka macht einen Substitutionsbedarf an diesen Stoffen und Blutspiegelkontrollen eventuell notwendig.
5. Eine weitere Verbesserung kann in der Darreichungsform erzielt werden.

Die Behebung der aufgelisteten Mängel gelingt überraschenderweise durch die Anwendung hochmolekularer alkylierter Polyethylenimine. Die nicht resorbierbaren Makromoleküle entfalten ihre Wirkung sowohl in löslicher Form, entsprechend der unvernetzten Struktur, als auch in unlöslichem Zustand als vernetzte Polymere.

Vernetzte Polyethylenimine werden in der US-Patentschrift 3 332 841 beschrieben. Die Vernetzung erfolgt u.a. über Alkylengruppen mit 2 bis 8 Kohlenstoffatomen, das Molekulargewicht der Ausgangspolymeren liegt zwischen 800 und 100 000. Zur Behandlung von vorübergehender Hyperacidität des Magens werden 0,25 bis 5 g pro Dosierungseinheit verabreicht. Es wird weder die Bindung von Gallensäure noch eine damit verbundene lipidsenkende Aktivität der vernetzten Polyethylenimine beschrieben, denn ohne Alkylierung besitzen die Polyethylenimine gegenüber den Gallensäuren je nach Typ keine oder nur unbedeutende Bindungskapazität. Durch die große potentielle Ladungsdichte kann durch die Alkylierung für die ausreichende Bindungskapazität und durch die Wahl der Substituenten von entsprechend hydrophil,/hydrophobem Charakter für die Affinität und Bindungsspezifität gesorgt werden.

In der Offenlegungsschrift DE-A-2 946 026 werden vernetzte alkylierte Polymere beschrieben, die durch Umsetzung einer stickstoffhaltigen Verbindung und einer bifunktionellen Halogenid- und/oder Epoxid- Verbindung erhältlich sind und aufgrund ihrer cholsäureadsorbierenden und lipoidsenkenden Wirkung als Mittel zur Bekämpfung von Arteriosklerose verwendet werden.

Die Erfindung betrifft unvernetzte und vernetzte alkylierte Polyethylenimine, dadurch gekennzeichnet, daß das Ausgangspolyethylenimin ein Molekulargewicht von 10 000 bis 10 000 000 aufweist, das Alkylierungsmittel die Formel I besitzt

R-X (I)

worin
- X: Chlor, Brom, Jod, CH₃-SO₂-O- oder 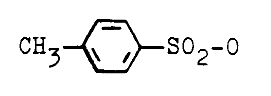 ist
und
- R: ein geradkettiger oder verzweigter C₁-C₃₀-Alkylrest ist, der gegebenenfalls substituiert ist mit einem mono- oder bicyclischen gesättigten Kohlenwasserstoff mit 5 bis 10 Ringkohlenstoffatomen, oder mit einem Phenylrest und, im Falle der vernetzten alkylierten Polyethylenimine das Vernetzungsmittel ein α,ω-Dihalogenalkan mit 2 - 10 Kohlenstoffatomen oder ein höher funktionelles Halogenalkan mit 2 - 10 Kohlenstoffatomen ist.

Das Verfahren zur Herstellung der erfindungsgemäß alkylierten Polyethyleniminderivate ist dadurch gekennzeichnet, daß man nach in der Polymerchemie üblichen Methoden ein Polyethylenimin mit einem Molekulkargewicht zwischen 10 000 und 10 000 000 mit einem Alkylierungsmittel der Formel R-X, worin X und R die angegebenen Bedeutungen haben, alkyliert und gegebenenfalls mit einem α,ω-Dihalogenalkan mit 2 - 10 Kohlenstoffatomen oder einem höher funktionellen Halogenalkan mit 2 - 10 Kohlenstoffatomen vernetzt.

Die Vernetzung kann vor oder nach der Alkylierung durchgeführt werden. Besonders bevorzugt ist die gleichzeitige Durchführung der Vernetzung und der Alkylierung.

Vorzugsweise werden Polyethylenimine mit einem Molekulargewicht über 100 000 eingesetzt.

In den Alkylierungsmitteln R-X ist X vorzugsweise Chlor oder Brom.

R ist vorzugsweise ein primärer Alkylrest. Falls die Alkylreste durch die genannten Ringsysteme substituiert sind, so sind diese vorzugsweise so angeordnet, daß sie über Spacer mit 1 bis 4 CH₂-Gruppen mit dem Polyethlyenimin verknüpft sind. Als monocyclischer gesättigter Substituent ist insbesonder der Cyclohexylrest geeignet. Ein geeigneter bicyclischer Kohlenwasserstoffrest ist z.B. Dekalin. Ein besonders geeignetes Alkylierungsmittel, dessen Alkylrest mit Phenyl substituiert ist, ist Benzylbromid. Als Alkylierungsmittel ohne Substituenten im Alkylrest kommt vorzugsweise Butylchlorid in Betracht.

Die Alkylierung kann in mehreren Teilschritten erfolgen. Dadurch besteht die Möglichkeit, verschiedene Substituenten am gleichen Polymeren zu fixieren.

Das Verhältnis des eingesetzten Alkylierungsmittels zu den Aminogruppen des Polyethylenimins beträgt 0,2:1 bis 5:1, vorzugsweise 0,5:1 bis 2:1.

Durch die Reaktion mit Alkylierungsmittel wird ein Teil der sekundären Aminogruppen der Kette in tertiäre und quartäre Strukturen überführt. Die Bildung von tertiären Aminogruppen wird bevorzugt.

Als Vernetzungsmittel eignen sich z.B. Di- und Trihalogenalkane, vorzugsweise α,ω-Dihalogenalkane wie z.B. 1,6-Dibromhexan und 1,10-Dibromdecan. Die Menge des Vernetzers beträgt vorzugsweise 2 - 25 Mol-%, bezogen auf das eingesetzte Alkylierungsmittel.

Die alkylierten Polyethylenimine adsorbieren körpereigene Säuren, insbesondere Gallensäure. Aufgrund dieser Eigenschaften sind sie in der Lage, erhöhte Cholesterinspiegel zu senken. Die alkylierten Polyethylenimine besitzen gegenüber Colestipol wesentlich günstigere gallensäureabsorbierende Eigenschaften wie aus den nachstend beschriebenen Versuchen erkennbar ist.

### 1. In vitro Versuche

1.1 Adsorptionsansatz mit einzelnen Gallensäuren Versuchsbedingungen: Volumen = 10 ml; Temperatur 37°C, Inkubation im Schüttelwasserbad, Dauer: 2 Stunden; Medium: isoton gepufferte physiologische Kochsalzlösung, pH 7.0; Gallensäure: 10 - 15 µMole; Adsorber (= erfindungsgemäße Verbindung oder Vergleichsverbindung): 10 - 100 mg.
   Die mit dem Adsorbat in Gleichgewicht stehende Gallensäure wird mittels enzymatischer Analyse bestimmt. Die Methoden über 3α-Hydroxy- oder 7α-Hydroxysteroiddehydrogenasen (EC 1.1.1.50 bzw. EC 1.1.1.159) wurden entsprechend der Beschreibung bei Bergmeyer (H.U. Bermeyer, Methoden der enzymatischen Analyse, 2. Aufl. (1970), S. 1824) bzw. der Produktinformation zu Prod.-Nr. H-9506 der Firma SIGMA CHEMICAL Co. (St. Louis, USA) durchgeführt. Aus der Differenz der Kontrollansätze ohne Adsorber und der Vollansätze wurde der gebundene Anteil an Gallensäuren errechnet. Die Versuche wurden unter Variation der vorgegebenen Gallensäurekonzentration oder der Adsorbermenge, seltener der Inkubationsdauer, pH oder Ionenstärke zur Charakterisierung des Adsorbers durchgeführt.
   Ein alkyliertes, vernetztes Polyalkylenimin gemäß Beispiel 2 zeigte eine qualitativ bessere Wirkung durch stärkere Cholatbindung als Colestipol, denn mit einer 2 mM Glykocholatlösung inkubiert adsorbierten 50 mg Colestipol unter 6 %, hingegen Polyimin gemäß Beispiel 2 74 % bis 76 % der Gallensäure.
1.2 Reversibilitätsprüfung. Das bei einem Ansatz wie unter 1.1 beschrieben aus dem Gleichgewicht entfernte Adsorbat wurde mit frischem gallensäurefreiem Medium nachinkubiert und die freigesetzte Gallensäure wie unter 1.1 bestimmt.
   In diesem Versuch konnte gezeigt werden, daß aus Adsorbaten mit alkylierten, vernetzten Polyethylenimin gemäß Beispiel 2 weniger Gallensäure wieder freigesetzt wurde, denn aus dem Adsorbat von 50 mg Colestipol mit 2 mM Glykocholat wurden wieder 57 % der Gallensäure freigesetzt, hingegen nur 4 % bis 5 % aus dem Adsorbat von 50 mg Verbindung gemäß Beispiel 2.
1.3 Adsorptionsansatz mit Gallensäuregemischen. Die unter 1.1 angegebenen Bedingungen wurden bezüglich Gallensäure so abgewandelt, daß simultan je 40 µMole der Taurokonjugate von Cholat, Chenodesoxycholat, Desoxycholat und Litocholat in den Ansatz gebracht wurden, wobei der Adsorber mit 20 - 100 mg zur Anwendung kam. Die individuelle Adsorptionsrate der Gallensäure wurde durch Auftrennung und Bestimmung mittels Hochdruckflüssigkeitschromatographie ermittelt (N. Parris, Analyt. Biochem. 100(1979) 260 - 263).
   Unter diesen Versuchsbedingungen zeigte ein erfindungsgemäßes alkyliertes Polyethylenimin gemäß Beispiel 1 eine größere Bindungsrate, denn von einem Gemisch von 4 taurokonjugierten Gallensäuren (jede mit 4 mM) banden 20 mg Colestipol 54 %, hingegen Polyimin gemäß Beispiel 1 83 %.

### In vivo Versuche

Junge männliche Wistar Ratten von etwa 200 g Körpergewicht wurden zu 6 Tieren in Gruppen geteilt und auf Standardfutter gehalten. Jeweils vor Versuchsbeginn, sowie 1 und 2 Wochen nach Versuchsbeginn wurden von den Tieren Kotproben für Analysen entnommen. Der Adsorber wurde, sofern wasserlöslich, als schwach sauer gepufferte Lösung mit der Magensonde täglich zu 100, 250 oder 500 mg/kg Körpergewicht 14 Tage lang verabreicht; als unlöslicher Stoff wurde der Adsorber mit 1 % Tylose^{(R)} (wasserlöslicher Celluloseether) als Vehikel suspendiert zu 250 oder 500 mg/kg Köerpergewicht täglich mit der Magensonde 14 Tage lang inkorporiert.

In Kotproben wurden die neutralen Steroide nach Homogenisierung mit Chloroform-Methanol 2:1 (v/v) extrahiert, der Extrakt hydrolysiert und das Hydrolysat mit Diethylether-Heptan 2:1 (v:v) extrahiert. Nach Verdampfen des Lösungsmittels wurde die Probe der gaschromatographischen Trennung und Auswertung (H-CH. Curtius und W. Bürgi, Z. Klin. Chemie 4 (1966) 38 - 42) unterworfen.
Die Untersuchungen der Kotproben zeigten folgendes:
2.1 War der Adsorber ein vernetztes, alkyliertes Polyethylenimin gemäß Beispiel 2 so wurde ein schnellerer Wirkungseintritt festgestellt, denn nach Verfütterung von 250 mg Adsorber/kg Körpergewicht täglich über 7 Tage an Ratten betrug die Mehrausscheidung unkonjugierter Gallensäure im Kot bei Colestipol 38 %, hingegen bei Verbindung gemäß Beispiel 2 115 %. Die Wirkung von Polyethylenimin erreicht man mit Colestipol erst nach 14 Tagen.
2.2 Die erwünschte Hemmung der bakteriellen Cholesterinumsetzung im Darm, verbunden mit einer Mehrausscheidung an Cholesterin, wird durch alkyliertes, vernetztes Polyethylenimin gemäß Beispiel 2 in stärkerem Maße hervorgerufen, denn unter gleichen experimentellen Bedingungen erhöht sich die Cholesterinausscheidung im Rattenkot durch Colestipol um 28 %, durch eine Verbindung gemäß Beispiel 2 um 89 %; gleichzeitig ändert sich die Koprostanolausscheidung durch Colestipol um +4 %, durch Verbindung gemäß Beispiel 2 aber um -30 %.

Aus den Versuchsergebnissen ist eindeutig erkennbar: Sowohl die unvernetzten wie auch die vernetzten alkylierten Polyethylenimine zeigen durch in vitro Adsorptionsversuche, daß gegenüber Colestipol
- die Quantität der gebundenen Gallensäure um 50 - 60 % erhöht ist (Beispiele 1 und 2)
- die Bindung von Cholat 10 - 12fach gesteigert wird (Beispiel 2)
- aus Gallensäure-Polymeradsorbaten die Wiederfreisetzungsrate 10 - 15fach niedriger ist (Beispiel 2).

In den Rattenversuchen wurde gezeigt, daß unvernetzte wie vernetzte alkylierte Polyethylenimine im Prüfbereich bis 500 mg/kg Körpergewicht täglich oral gegeben symptomlos vertragen werden. Vorteile gegenüber gleichdosierten Colestipolgaben konnten dadurch gezeigt werden, daß
- ein schnellerer Wirkungseintritt stattfindet, wodurch nach einwöchiger Anwendung die Gallensäuremehrausscheidung verdreifacht war
- die Cholesterinelimination dreifach erhöht war
- die bakterielle Umsetzung von Cholesterin im Darm gebremst und dadurch die Produktion und Ausscheidung von Koprostanol signifikant (= 30 %) verringert wurde (Beispiel 2)

Die Verbindungen eignen sich aufgrund ihrer Eigenschaften zur Verwendung als Arzneimittel, insbesondere zur Senkung erhöhter Lipidspiegel. Die Erfindung betrifft daher auch die Verwendung als Antihyperlipidämikum sowie pharmazeutische Mittel. In den pharmazeutischen Mitteln können die Polyethylenimine auch in Form physiologisch verträglicher Salze mit Säuren vorliegen.

Ein besonderer Vorteil ist die Verwendung vernetzter alkylierter Polyethylenimine. Die vernetzten Produkte können keine Stoffe an ihre Umgebung abgeben. Dies ist von Bedeutung für die Entwicklung eines nicht toxischen Materials.

Die täglich zu verabreichende Dosis beträgt vorzugsweise 1,0 bis 10,0 g, insbesondere 5 g. Sie kann auf mehrere Einzeldosen verteilt werden.

Die Verbindungen können als solche oder nach Zusatz von üblichen Hilfsstoffen in oral verabreichbare Zubereitungsformen überführt werden, wie z.B. Tabletten, Kapseln, Sirupe, wäßrige Lösungen, Suspensionen usw. Hierbei kann es zweckmäßig sein, in fester Form anfallende Wirkstoffe zunächst z.B. durch Feinmahlung auf eine gewünschte Partikelgröße zu bringen. Geeignete Hilfsstoffe sind z.B. Lactose, Stärke, Gelatine, Talkum usw. Die Herstellung von Tabletten erfolgt z.B. durch Feuchtgranulation und anschließender Verpressung.

Darüberhinaus können die alkylierten Polyethylenimine auch in Nahrungsmittel wie Brot, Fruchsäfte usw. eingearbeitet werden oder zusammen mit Nahrungsmitteln eingenommen werden.

Die Verbindungen können auch in Kombination mit weiteren Wirkstoffen zur Anwendung gelangen. Als weitere Wirkstoffe eignen sich z.B. HMG-CoA-Reduktasehemmer, Vitamine, Geriatrika und Antidiabetika.

Die nachfolgenden Beispiele sollen die Erfindung erläutern:

### Beispiel 1

In einem 4 l Reaktionskolben werden 44,3 g Polyethylenimin (Mol.Gew. etwa 1 000 000) in 1,05 l H₂O gelöst; 191,4 g Butylchlorid werden zugegeben und unter starkem Rühren (500 U/Min) 24 Stunden zum Rückfluß erhitzt. Es bildet sich eine trübe, viskose Reaktionsmischung. Zu dieser Mischung wird nach dem Abkühlen 1 l 2n NaOH-Lösung zugegeben und der Ansatz nochmals 24 Stunden zum Rückfluß gebracht. Nach dem Abkühlen bilden sich 2 Phasen. Die organische Phase wird abgetrennt und vom Lösungsmittel im Vakuum befreit. Die Auswaage an Feststoff beträgt 75,4 g.
Das Produkt besitzt einen Alkylierungsgrad von etwa 50 % und ist nach Zugabe äquivalenter Mengen Säure wasserlöslich.

### Beispiel 2

In einem 4 l Reaktionskolben werden 44,3 g (1 Mol) Polyethylenimin in 1,05 l H₂O gelöst; 186,6 g Butylchlorid (2 Mol) und 48,8 g (0,2 Mol) 1,6-Dibromhexan werden zugegeben und unter starkem Rühren (500 U/Min.) 24 Stunden zum Rückfluß erhitzt. Es bildet sich eine trübe, viskose Reaktionsmischung. Zu dieser Mischung wird nach dem Abkühlen 1 l 2n NaOH-Lösung zugegeben und der Ansatz nochmals 24 Stunden zum Rückfluß gebracht. Nach dem Abkühlen der Reaktionsmischung wird der Feststoff abgesaugt, neutral gewaschen und getrocknet. Die Auswaage beträgt 87,27 g. Das Produkt ist in Wasser unlöslich und quellbar in Methanol.

Das Verhältnis von Ausgangsverbindung zu Alkylierungsmittel und Vernetzungsmittel kann in gewissen Grenzen variiert werden. Unter den angegebenen Reaktionsbedingungen erhält man dann Produkte mit anderem Alkylierungs- und Vernetzungsgrad.

### Beispiel 3

Das Reaktionsgemisch aus 4,3 g Polyethylenimin, 100 ml Wasser und 29,3 g 1-Chlor-2-cyclohexylethan wird 24 Stunden zum Rückfluß erhitzt, wobei sich eine gelbliche trübe Mischung ergibt. Nach Zugabe von 100 ml 2n NaOH-Lösung wird die Mischung erneut 24 Stunden zum Rückfluß erhitzt. Die Reaktionsmischung bildet ein 2-Phasensystem. Die organische Phase wird abgetrennt, am Rotationsverdampfer vom Lösungsmittel befreit. Man erhält 15,34 g eines hochviskosen Materials.

### Beispiel 4

Eine Mischung aus 88,6 g Polyethylenimin (50-proz. in Wasser; ≙ 1 Mol), 186,6 g n-Butylchlorid und 48,8 g 1,6-Dibromhexan in 2 l Wasser wird in einem Rühr- oder Schüttelautoklaven unter 10 bar Stickstoff 24 Stunden auf 80°C erwärmt. Man ersetzt das Schutzgas durch 7 bar Ammoniak und erwärmt nochmals 24 Stunden auf 80°C.

Nach dem Abkühlen der Reaktionsmischung wird der entstandene Niederschlag abgesaugt und mit Wasser neutral gewaschen. Das Produkt wird mit Methanol gewaschen und in einer Säule mit ca. 2,5 l Methanol, ca. 1,5 l 2 N Essigsäure, ca. 2 l ∼2 N Ammoniakwasser und schließlich ca. 2 l methanol eluiert. Nach dem Absaugen wird das Produkt bei maximal 50°C im Vakuum getrocknet.
Ausbeute 78 g.
Das Produkt ist in diversen Lösungsmitteln quellbar, jedoch unlöslich.

## Patentansprüche (Patentansprüche für folgende(n) Vertragsstaat(en): AT, BE, CH, DE, DK, FR, GB, IT, LI, LU, NL, SE)

1. Unvernetzte und vernetzte alkylierte Polyethylenimine, erhältlich durch Umsetzung eines Polyethylenimins, das ein Molekulargewicht von 10 000 bis 10 000 000 aufweist, mit einem Alkylierungsmittel der Formel I
R-X (I)
worin
X Chlor, Brom, Jod, CH₃-SO₂-O- oder 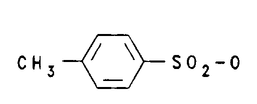 ist
und
R ein geradkettiger oder verzweigter C₁-C₃₀-Alkylrest ist, der gegebenenfalls substituiert ist mit einem mono- oder bicyclischen gesättigten Kohlenwasserstoff mit 5 bis 10 Ringkohlenstoffatomen, oder mit einem Phenylrest
und, im Falle der vernetzten alkylierten Polyethylenimine mit einem α, ω-Dihalogenalkan mit 2 - 10 Kohlenstoffatomen oder einem höher funktionellen Halogenalkan mit 2 - 10 Kohlenstoffatomen als Vernetzungsmittel, zur Anwendung als gallensäureadsorbierendes Arzneimittel.

2. Pharmazeutisches Präparat, dadurch gekennzeichnet, daß es eine Verbindung gemäß Anspruch 1 oder deren physiologisch verträgliches Salz mit einer Säure enthält.

3. Verfahren zur Herstellung eines pharmazeutischen Präparates, dadurch gekennzeichnet, daß man eine Verbindung gemäß Anspruch 1 in eine geeignete Darreichungsform überführt.

4. Verwendung einer Verbindung gemäß Anspruch 1 als Antihyperlipidämikum.

5. Verwendung von Verbindungen gemäß Anspruch 1 als Zusatz in Lebensmitteln und Fruchtsäften.

## Patentansprüche (Patentansprüche für folgende(n) Vertragsstaat(en): ES, GR)

1. Verfahren zur Herstellung eines pharmazeutischen Präparates, dadurch gekennzeichnet, daß man ein unvernetztes oder vernetztes alkyliertes Polyethyleniminderivat durch Alkylierung von Polyethylenimin mit einem Molekulargewicht von 10 000 und 10 000 000 mit einem Alkylierungsmittel der Formel I
R-X (I)
worin
X Chlor, Brom, Jod, CH₃-SO₂-O- oder 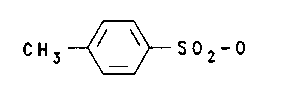 ist
und
R ein geradkettiger oder verzweigter C₁-C₃₀-Alkylrest ist, der gegebenenfalls substituiert ist mit einem mono- oder bicyclischen gesättigten Kohlenwasserstoff mit 5 bis 10 Ringkohlenstoffatomen, oder mit einem Phenylrest
und gegebenenfalls Vernetzung mit einem α, ω-Dihalogenalkan mit 2 - 10 Kohlenstoffatomen oder einem höher funktionellen Halogenalkan mit 2 - 10 Kohlenstoffatomen herstellt, und, gegebenenfalls nach Überführung in ein physiologisch verträgliches Salz, in einem weiteren Verfahrensschritt in eine geeignete Darreichungsform überführt.

2. Verfahren gemäß Anspruch 1, dadurch gekennzeichnet, daß eine oder mehere der folgenden Maßnahmen a) bis f) eingehalten werden:
a) Polyethylenimine mit einem Molekulargewicht über 100 000 eingesetzt werden,
b) in den Alkylierungsmitteln R-X, X Chlor oder Brom ist,
c) in den Alkylierungsmitteln R ein primärer Alkylrest ist, der gegebenenfalls substituiert ist mit einem Cyclohexyl-, Dekalin- oder Phenylrest, wobei diese Substituenten so angeordnet sind, daß sie über einen Spacer mit 1 bis 4 CH₂-Gruppen mit dem Polyethylenimin verknüpft sind,
d) das Verhältnis des eingesetzten Alkylierungsmittels zu den Aminogruppen des Polyethylenimins 0,5:1 bis 2:1 beträgt,
e) das Vernetzungsmittel 1,6-Dibromhexan oder 1,10-Dibromdecan ist,
f) Alkylierung und Vernetzung gleichzeitig durchgeführt werden,
und daß in dem weiteren Verfahrensschritt die erhaltene Verbindung in eine als Antihyperlipidämikum geeignete Darreichungsform überführt wird.

## Claims (Claims for the following Contracting State(s): AT, BE, CH, DE, DK, FR, GB, IT, LI, LU, NL, SE)

1. A non-crosslinked or crosslinked alkylated polyethylenimine, obtainable by reacting a polyethylenimine which has a molecular weight of 10,000 to 10,000,000, with an alkylating agent of the formula I
R-X (I)
in which
X is chlorine, bromine, iodine, CH₃-SO₂-O- or 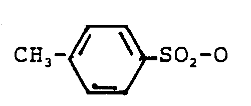 and
R is a straight-chain or branched C₁-C₃₀-alkyl radical which is optionally substituted by a mono-or bicyclic saturated hydrocarbon having 5 to 10 ring carbon atoms, or by a phenyl radical
and, in the case of the crosslinked alkylated polyethylenimines, with an α,ω-dihaloalkane having 2-10 carbon atoms or a higher functionalized haloalkane having 2-10 carbon atoms as crosslinking agent, for use as a pharmaceutical which adsorbs bile acid.

2. A pharmaceutical preparation which contains a compound as claimed in claim 1 or its physiologically tolerated salt with an acid.

3. A method for the production of a pharmaceutical preparation, which comprises converting a compound as claimed in claim 1 into a suitable form for administration.

4. The use of a compound as claimed in claim 1 as an antihyperlipidemic agent.

5. The use of compounds as claimed in claim 1 as an additive in foodstuffs and fruit juices.

## Claims (Claims for the following Contracting State(s): ES, GR)

1. A method for the production of a pharmaceutical preparation, which comprises preparing a non-crosslinked or crosslinked alkylated polyethylenimine derivative by alkylation of a polyethylenimine with a molecular weight of 10,000 to 10,000,000, using an alkylating agent of the formula I
R-X (I)
in which
X is chlorine, bromine, iodine, CH₃-SO₂-O- or 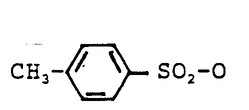 and
R is a straight-chain or branched C₁-C₃₀-alkyl radical which is optionally substituted by a mono-or bicyclic saturated hydrocarbon having 5 to 10 ring carbon atoms, or by a phenyl radical
and, if desired, crosslinking with an α,ω-dihaloalkane having 2-10 carbon atoms or a higher functionalized haloalkane having 2-10 carbon atoms and, if desired after conversion into a physiologically tolerated salt, converting the product into a suitable form for administration, in a further process step.

2. The process as claimed in claim 1, wherein one or more of the following measures a) to f) are observed:
a) polyethylenimines having a molecular weight above 100,000 are employed,
b) in the alkylating agents R-X, X is chlorine or bromine,
c) in the alkylating agents, R is a primary alkyl radical which is optionally substituted by a cyclohexyl, decalin or phenyl radical, where these substituents are arranged such that they are linked to the polyethylenimine via a spacer having 1 to 4 CH₂ groups,
d) the ratio of the alkylating agent employed to the amino groups of the polyethylenimine is 0.5:1 to 2:1,
e) the crosslinking agent is 1,6-dibromohexane or 1,10-dibromodecane,
f) alkylation and crosslinking are carried out simultaneously,
and wherein, in the further process step, the resulting compound is converted into a suitable form for administration as antihyperlipidemic agent.

## Revendications (Revendications pour l'(les) Etat(s) contractant(s) suivant(s): AT, BE, CH, DE, DK, FR, GB, IT, LI, LU, NL, SE)

1. Polyéthylène-imines alkylées, réticulées ou non réticulées, pouvant être obtenues par la réaction d'une polyéthylène-imine qui présente une masse moléculaire de 10 000 à 10 000 000, avec un agent d'alkylation de formule I
R-X (I)
dans laquelle
X est un atome de chlore, de brome ou d'iode ou le groupe CH₃-SO₂-O- ou 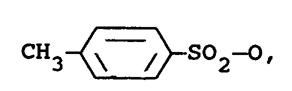 et
R est un radical alkyle en C₁-C₃₀ à chaîne droite ou ramifiée, qui est éventuellement substitué par un radical hydrocarboné saturé mono- ou bicyclique, ayant de 5 à 10 atomes de carbone formant le cycle, ou par le radical phényle,
et, dans le cas des polyéthylène-imines alkylées et réticulées, l'agent de réticulation est un α,ω-dihalogénoalcane ayant de 2 à 10 atomes de carbone ou un halogénoalcane à fonctionnalité supérieure, ayant de 2 à 10 atomes de carbone, pour utilisation en tant que médicaments adsorbant les acides biliaires.

2. Composition pharmaceutique, caractérisée en ce qu'elle contient un composé selon la revendication 1 ou un de ses sels physiologiquement acceptable avec un acide.

3. Procédé pour la préparation d'une composition pharmaceutique, caractérisé en ce que l'on met sous une forme d'administration appropriée un composé selon la revendication 1.

4. Utilisation d'un composé selon la revendication 1 en tant qu'antihyperlipidémiant.

5. Utilisation de composés selon la revendication 1 comme additifs dans des produits alimentaires et des jus de fruits.

## Revendications (Revendications pour l'(les) Etat(s) contractant(s) suivant(s): ES, GR)

1. Procédé pour la préparation d'une composition pharmaceutique, caractérisé en ce que l'on prépare un dérivé de polyéthylène-imine alkylé, réticulé ou non réticulé, par alkylation d'une polyéthylène-imine ayant une masse moléculaire de 10 000 à 10 000 000, avec un agent d'alkylation de formule I
R-X (I)
dans laquelle
X est un atome de chlore, de brome ou d'iode ou le groupe CH₃-SO₂-O- ou 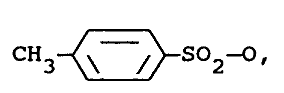 et
R est un radical alkyle en C₁-C₃₀ à chaîne droite ou ramifiée, qui est éventuellement substitué par un radical hydrocarboné saturé mono- ou bicyclique, ayant de 5 à 10 atomes de carbone formant le cycle, ou par le radical phényle,
et, éventuellement réticulation avec un α,ω-dihalogénoalcane ayant de 2 à 10 atomes de carbone ou un halogénoalcane à fonctionnalité supérieure, ayant de 2 à 10 atomes de carbone, et, éventuellement après conversion en un sel physiologiquement acceptable, dans une autre étape de processus, on le met sous une forme d'administration appropriée.

2. Procédé selon la revendication 1, caractérisé en ce que l'on respecte une ou plusieurs des dispositions a) à f) suivantes:
a) on utilise des polyéthylène-imines ayant une masse moléculaire supérieure à 100 000,
b) dans les agents d'alkylation R-X, X est le chlore ou le brome,
c) dans les agents d'alkylation, R est un radical alkyle primaire qui est éventuellement substitué par un radical cyclohexyle, décaline ou phényle, ces substituants étant disposés de manière à être liés à la polyéthylène-imine par l'intermédiaire d'un espaceur comportant 1 à 4 groupes CH₂,
d) le rapport de l'agent d'alkylation utilisé aux groupes amino de la polyéthylène-imine va de 0,5:1 à 2:1,
e) l'agent de réticulation est le 1,6-dibromohexane ou le 1,10-dibromodécane,
f) l'alkylation et la réticulation sont effectuées simultanément,
et en ce que, dans l'autre étape de processus, on met le composé obtenu sous une forme d'administration appropriée, en tant qu'antihyperlipidémiant.
